(19) Europäisches Patentamt
European Patent Office
Office européen des brevets

(11) **EP 4 539 729 B1**

(12) **EUROPEAN PATENT SPECIFICATION**

(45) Date of publication and mention
of the grant of the patent:
**29.04.2026  Bulletin 2026/18**

(21) Application number: **23733417.2**

(22) Date of filing: **12.06.2023**

(51) International Patent Classification (IPC):
**A61B 5/00** *(2006.01)*  **H03M 7/30** *(2006.01)*

(52) Cooperative Patent Classification (CPC):
**A61B 5/0024; A61B 5/7232; H03M 7/3062;**
A61B 2560/0209

(86) International application number:
**PCT/GB2023/051520**

(87) International publication number:
**WO 2023/242541 (21.12.2023 Gazette 2023/51)**

(54) **BODY SENSOR NETWORK**

KÖRPERSENSORNETZWERK

RÉSEAU DE CAPTEURS CORPORELS

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **14.06.2022  GB 202208740**

(43) Date of publication of application:
**23.04.2025  Bulletin 2025/17**

(73) Proprietor: **University of Essex Enterprises
Limited
Colchester
Essex CO4 3SQ (GB)**

(72) Inventors:
• **ABOLGHASEMI, Vahid**
  **Colchester Essex CO4 3SQ (GB)**
• **ANISI, Mohammad Hossein**
  **Colchester Essex CO4 3SQ (GB)**

(74) Representative: **Slingsby Partners LLP
1 Kingsway
London WC2B 6AN (GB)**

(56) References cited:
US-A1- 2019 125 271

• GHOSH SUSHMITA ET AL: "Learning-based
Smart Sensing for Energy-Sustainable WSN",
ICC 2021 - IEEE INTERNATIONAL CONFERENCE
ON COMMUNICATIONS, IEEE, 14 June 2021
(2021-06-14), pages 1 - 6, XP033953623, DOI:
10.1109/ICC42927.2021.9500317
• CHEN HSIEH-CHUNG ET AL: "Compressive
wireless pulse sensing", 2015 INTERNATIONAL
CONFERENCE ON COLLABORATION
TECHNOLOGIES AND SYSTEMS (CTS), IEEE, 1
June 2015 (2015-06-01), pages 5 - 11,
XP033196684, ISBN: 978-1-4673-7647-1,
[retrieved on 20150819], DOI: 10.1109/
CTS.2015.7210388
• DROR BARON ET AL: "Distributed Compressive
Sensing", ARXIV.ORG, CORNELL UNIVERSITY
LIBRARY, 201 OLIN LIBRARY CORNELL
UNIVERSITY ITHACA, NY 14853, 22 January 2009
(2009-01-22), XP080358356

**Description**

BACKGROUND OF THE INVENTION

There are many advantages to being able to wirelessly monitor a person's physiological data (e.g., heart rate, blood pressure, respiratory rate) during their day-to-day life. Keeping track of a person's heart rate and blood pressure, for example, might enable signs of a developing health condition to be identified. In some examples, it may be useful to be able to monitor a patient's vital signs after they have been released from hospital following an operation. Patients may be released from hospital sooner after an operation if health care professionals felt confident that the patient's physiological parameters could be reliably and accurately monitored at home with an on-body device. This in turn can help to free-up hospital beds and resources.

Devices that can monitor a user's physiological parameters in day-to-day life often suffer from being bulky, expensive, and require re-charging frequently (e.g., every few days). This is because the process of obtaining and processing body sensor data is energy intensive, computationally intensive, and requires large amounts of storage. However, the body sensors themselves usually have a very limited battery life and have limited storage and computational capabilities. In particular, transmitting data wirelessly consumes a substantial proportion of the energy of the sensors. This challenges both the quality of the communicated information and the processing resources available at the sensor. Furthermore, needing to recharge the sensors frequently will reduce the efficacy of the monitoring device as the user will be frequently taking the sensors off. This increases the likelihood that signs of a critical condition may be missed and can be dangerous for users who need near constant monitoring for their health conditions. Furthermore, the user may feel less free to move around if they know that the sensors cannot last a long time away from charging facilities.

Recent work in the field has recognised the benefit of providing near continuous monitoring of a user's biometric data.

The paper *"A Sensor Fusion Wearable health-monitoring System with Haptic Feedback"* by F. Sanfilippo and K. Y. Pettersen, 2015 11th International Conference on Innovations in Information Technology (IIT), Dubai, 2015, pp. 262-266, discloses a preliminary working prototype of a wearable integrated health monitoring system that provides the user with haptic feedback and allows the user to report an emergency. One application discussed is for monitoring the vitals of workers such as offshore operators. The monitored data is sent wirelessly to cloud-based data storage, however, the energy constraints on the system are not discussed.

Rinicare have developed the STABILITY system which monitors a patient's condition post-surgery. This provides a personalised score from all currently recorded patient data that predicts the likelihood of an improvement or deterioration in the patient's condition. However, this system does not consider the energy efficiency of capturing physiological data.

*ADAMM intelligent asthma monitoring* is a wireless wearable device that adheres to the skin around the chest and monitors respiratory rate, oxygen levels, pulse, blood pressure and body temperature. It uses machine learning to analyse the user's data to alert users to risks of asthma attacks. The device is intended to be recharged overnight in a cradle next to the user's bed.

US 2019/125271 discloses a body area network comprising wireless sensors - in particular PPG sensor, ECG sensor, 3-D accelerometer - that sense one or more vital signs and communicate them to an aggregator. Different applications of compressed sensing (CS) are proposed concerning low power and robust sensors in the body area networks for healthcare and fitness applications. It is demonstrated that pulse oximeter sensor acquisition power may be significantly reduced while not compromising its utility in clinical applications.

*"Learning-based Smart Sensing for Energy-Sustainable WSN"* by Sushmita Gosh et. al discloses a learning-based adaptive sampling framework that explores the sparsity in the time series data and finds optimal sampling instants for the next measurement cycle.

*"Compressive Wireless Pulse Sensing"* by Hsieh-Chung Chen et. al discloses a compressive sensing-based approach to wireless devices that exploits sparsity of signal to reduce power consumption for both sensing and transmission.

*"Distributed Compressive Sensing"* by Baron Dror et. al discloses a theory for distributed compressive sensing (DCS) that enables distributed coding algorithms for multi-signal ensembles that exploit both intra and inter-signal correlation structures.

SUMMARY OF THE INVENTION

There is provided a system for adaptively capturing physiological data using compressive sensing, the system comprising:

> a plurality of sensors each configured to sample a respective physiological signal according to a sampling pattern defined by a respective sensing matrix; and

a processor configured to:

receive sampled data from the plurality of sensors;
approximate the physiological signals from the sampled data;
identify one or more correlations between the approximated physiological signals;
update one or more of the sensing matrices in dependence on the one or more detected correlations; and
transmit updated one or more sampling patterns defined by the updated sensing matrices to the respective sensors of the plurality of sensors for use in sampling the respective physiological signals.

The processor may be configured to approximate each physiological signal from the sampled data by, using the respective sensing matrix, solving for a sparse representation of the physiological signal by applying compressive sensing to the respective sampled data.

The processor may be configured to approximate each physiological signal from the sampled data using interpolation or principal component analysis.

The processor may be configured to update each sensing matrix by determining a respective basis that sparsely represents the respective approximated signal and determine a respective updated sensing matrix that is incoherent with the determined basis.

Solving for a sparse representation of each physiological signal may be performed using the respective basis previously determined to sparsely represent the previously approximated physiological signal.

The processor may be configured to infer changes to the respective physiological signal based on a measure of change of the respective basis each time the respective sensing matrix is updated.

For each physiological signal, the respective basis may be determined using a sparse coding algorithm that finds a sparser basis with which to represent the respective approximated signal, wherein the approximated signal is used as training data for the sparse coding algorithm.

The identified correlations may be used as an additional constraint in the sparse coding algorithm.

Compressive sensing may be applied using an $l_1$-norm so as to solve for the sparse representation of the physiological signal.

The processor may identify a measure of correlation between two or more physiological signals.

The processor may update one or more sensing matrices in dependence on whether the measure of correlation between two or more physiological signals deviates from an expected value by more than a predefined threshold.

The processor may update one or more sensing matrices in dependence on whether the rate of change of the measure of correlation between two or more physiological signals deviates from an expected value by more than a predefined threshold.

The processor may update one or more sensing matrices to sample less frequently if the measure of correlation between the respective physiological signals exceeds a predefined threshold.

The processor may update one or more sensing matrices to sample more frequently if the measure of correlation between the respective physiological signals falls below a predefined threshold.

The measure of correlation may be one or more of: Pearson's correlation coefficient, Spearman's rank correlation coefficient, or a regression line.

One of the sensors may be an accelerometer configured to measure movement of the user.

The processor may be configured to compare correlations between the accelerometer data and the other physiological signals measured by the system so as to correct for artifacts in the physiological signals caused by human movement during sampling of the sampled data.

For each physiological signal, the basis may be determined using the K-SVD machine learning algorithm.

The physiological signals may be sparse signals such that each physiological signal of length N can be represented as a linear combination of K basis vectors, wherein K << N.

The processor may comprise a receiving device configured to attach to a user. The receiving device may receive sampled data from each of the plurality of sensors.

There is provided a method for adaptively capturing physiological data using compressive sensing, the method comprising:

sampling physiological signals at a plurality of sensors, each sensor being configured to sample a respective physiological signal according to a respective sampling pattern defined by a respective sensing matrix; and
approximating the physiological signals from data sampled by the sensors;
identifying one or more correlations between the approximated physiological signals;
updating one or more of the sensing matrices in dependence on the one or more identified correlations; and
transmitting updated one or more sampling patterns defined by the respective updated sensing matrices to respective sensors for use in sampling the respective physiological signals.

Approximating each physiological signal from the sampled data may comprise, using the respective sensing matrix, solving for a sparse representation of the physiological signal by applying compressive sensing to the respective sampled data.

Approximating each physiological signal from the sampled data may comprise using interpolation or principal component analysis.

Updating each sensing matrix may comprise determining a respective basis that sparsely represents the respective approximated signal and determining a respective updated sensing matrix that is incoherent with the determined basis.

Solving for a sparse representation of each physiological signal may be performed using the respective basis previously determined to sparsely represent the previously approximated physiological signal.

The method may further comprise identifying changes to the respective physiological signal based on a measure of change of the respective basis each time the respective sensing matrix is updated.

Determining a respective basis for each physiological signal may comprise using a sparse coding algorithm that finds a sparser basis with which to represent the respective approximated signal. The approximated signal may be used as training data for the sparse coding algorithm.

The identified correlations may be used as an additional constraint in the sparse coding algorithm.

Identifying one or more correlations may comprise determining a measure of correlation between two or more approximated physiological signals.

One or more of the sensing matrices may be updated in dependence on whether the measure of correlation between two or more physiological signals deviates from an expected value by more than a predefined threshold.

One or more sensing matrices may be updated in dependence on whether the rate of change of the measure of correlation between two or more physiological signals deviates from an expected value by more than a predefined threshold.

One or more sensing matrices may be updated to increase the sampling frequency of one or more respective physiological signals if the measure of correlation between two or more physiological signals exceeds a predefined threshold.

One or more sensing matrices may be updated to decrease the sampling frequency of one or more respective physiological signals if the measure of correlation between two or more physiological signals falls below a predefined threshold.

Any one or more feature of any aspect above may be combined with any other aspect. These have not been written out in full here merely for the sake of brevity. The features of the above system claims may be implemented as equivalent method claims and vice versa.

## BRIEF DESCRIPTION OF THE DRAWINGS

Figure 1 shows an example system 1 comprising a plurality of sensors 2 and a processor 3.

Figures 2a and 2b show examples of sampling patterns.

Figure 3 shows a visualisation of the relationships between **y** sampled data, **x** the original signal, $\Phi$ the sensing matrix, the basis $\Psi$ and the sparse signal s.

Figure 4 shows some example steps in a method for capturing physiological data using compressive sensing.

Figure 5 shows some example steps in a method for adaptively capturing physiological data using compressive sensing.

## DETAILED DESCRIPTION OF THE INVENTION

The inventors of this application have recognised that there is a large amount of redundancy in a user's physiological data when the user is being monitored constantly. A large amount of the data being transmitted from body sensors will convey essentially the same information and so is often not particularly useful. For example, when a user is sleeping, their blood pressure is likely to remain relatively constant throughout the night. If this is the case, then there is no need to take a measurement of the user's blood pressure every minute. Instead, taking a measurement every half an hour would produce the same picture of the user's state.

Reducing the number of measurements taken by each sensor will increase the battery life of the body sensors in two-ways. Firstly, the act of taking the measurement uses battery power. Secondly, and more importantly, the act of wirelessly transmitting the sampled data is very energy intensive. So, it would be advantageous if the sensors could take fewer measurements so that less data is wirelessly transmitted and/or so that the sensors transmit data less frequently. However, there is a risk, when sampling less frequently, that vital information might be missed by the sensors. So, there is a balance to be struck in taking as few measurements as possible whilst not losing important information from the user's physiological signals.

Furthermore, it would be advantageous if the sensors were able to adapt the number of samples taken depending on the state of the user and the user's activity history. For example, some users may have a more erratic heart rate than other users, and may need their heart rate to be monitored more closely than other users. When the users need the most monitoring may be dependent, for example, on the time of day or their physical state (e.g. whether they are exercising or at rest). There are likely to be patterns in a user's day that correspond to changes in their physiological state. In general, it would be advantageous if the sensors were able to automatically adapt the frequency of sampling to best suit the user.

This application uses compressive sensing technology to reduce the energy consumption of the sensors in a wireless body sensor network (WBSN) by reducing the number of samples taken and wirelessly transmitted at each sensor, without compromising the quality of information obtained by the sensors.

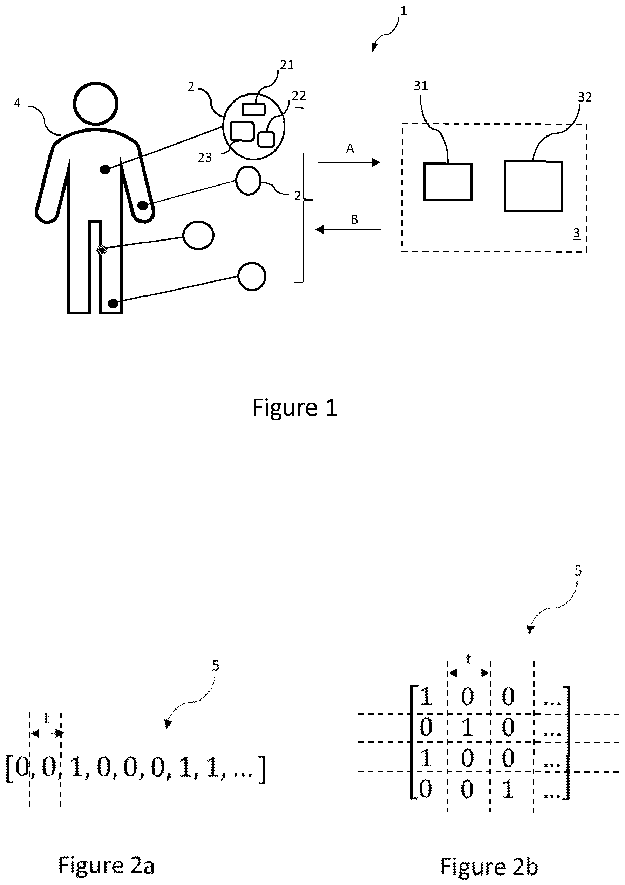

Figure 1 shows an example system 1 comprising a plurality of sensors 2 and a processor 3. The system 1 is an example of a wireless body sensor network. Each sensor 2 samples a physiological signal. A physiological signal is produced from measuring physiological data over time. For example, heart rate may be the physiological data being sampled, and the physiological signal is produced from measuring that heart rate over a certain period of time. Physiological data (herein equivalently referred to as physiological signals) may be defined as data relating to the body of the user. The user is likely to be a human but it may be any animal that has measurable physiological data. Examples of physiological data are heart rate, blood pressure, skin temperature, respiratory rate, and limb or body movement.

The plurality of sensors 2 may be attached to a user 4 at different positions on the user's body (as shown in figure 1) so that different physiological signals of the user can be measured. The sensors are designed to be continuously worn by the user during day-to-day life.

Each sensor 2 is arranged to measure a physiological signal. One or more of the sensors may measure different physiological signals. Some sensors may measure the same physiological signal as other sensors. This may help improve the accuracy of the physiological signal being measured. Some sensors may measure several physiological signals at one time. The specific physiological signal (or signals) sampled by each sensor may depend on where each sensor is positioned on the body and what physiological signal each sensor is designed to measure. For example, a first sensor may measure the user's heart rate - e.g. at a chest strap adapted to measure the electrical activity of the heart. A second sensor may measure the user's blood oxygen levels - e.g. over an artery at the wrist. A third sensor may measure the user's body movements - e.g. at a limb of the user. One or more of the first, second and third sensors could share the same sensor hardware and/or be located at the same point on the user's body - for example, sensor hardware at the wrist could comprise the first sensor for measuring heart rate and the second sensor for measuring blood oxygen.

Figure 1 shows an example sensor 2 comprising a measurement unit 21, a transceiver unit 22 and a memory unit 23. The measurement unit 21 enables the sensor to capture the respective physiological signal. For example, the measurement unit 21 may be a heart rate monitor or blood oxygen monitor. The measurement unit 21 may vary between sensors depending on what physiological signal each sensor is adapted to sample. The sensors 2 may comprise a transceiver unit 22. The transceiver unit 22 transmits the sampled data (e.g. to the processor) and receives data (e.g. from the processor). In some examples, the sensors 2 may optionally comprise a memory unit 23. The memory unit 23 may store sampled data until the data is transmitted to the processor.

The sensors may optionally perform some simple signal processing prior to transmitting the sampled data to the processor. The transmitted sampled data may comprise the raw measurements captured by the sensor and/or values derived from such raw measurements. For example, the sensors may average one or more measurements prior to transmission. In addition to the sensors 2 shown in figure 1, the system 1 may further include conventional sensors for capturing physiological data which do not sample according to sampling patterns in the manner described herein.

The data sampled by the plurality of sensors is transmitted to the processor 3, as signified by the arrow A in figure 1. The processor transmits sampling patterns to the sensors 2, as signified by the arrow B in figure 1.

A sampling pattern describes when a measurement (equivalently referred to as a sample) is to be taken by a sensor. Each sensor receives a respective sampling pattern that determines when that sensor is to take samples. For example, a sampling pattern may determine a frequency at which the sensor takes samples - e.g. every 30 seconds. Generally, the sampling pattern may indicate to the respective sensor in any suitable manner when it is to take a measurement. The sampling pattern need not cause the sensor to take a sample at a uniform rate (e.g. every 30 seconds).

A sampling pattern may indicate when measurements are to be taken within some window of time represented by the sampling pattern. For example, a sampling pattern may represent, for a sequence of time slots, whether or not samples are to be taken in each of those time slots. For example, the sampling pattern may comprise a set of randomly populated binary values, each value indicating whether or not a measurement is to be taken in the respective time slot. In some cases, a sampling pattern may determine that the sensor is not to take any samples.

Figure 2 shows two examples of sampling patterns.

Figure 2a shows an example sampling pattern 5 that may be received by a sensor. The sampling pattern shown in figure 2a is an array of values comprising 0s and 1s. The sampling pattern in the example shown in figure 2a is populated with random binary values, 0 or 1. Each entry in the sampling pattern determines whether the sensor is to take a sample or not. In figure 2a and figure 2b, each entry (0 or 1) corresponds to each time slot of a sequence, as indicated by the label "t".

Figure 2b shows another example sampling pattern 5 expressed in matrix form. When expressed as a matrix, the length of the sequence (e.g., the length of a row) may define a particular time, such as an hour. This may be determined by the processor.

The sensor reads a value from the sampling pattern at each time slot (e.g. either 0 or 1) and takes a measurement or not depending on the value. If the sampling pattern is expressed as a matrix, at the next time sequence (e.g. in next row of the matrix), the sensor reads values from the next row in the matrix.

The time base of each time slot (e.g., the amount of time between the sensor reading the next entry, denoted by "t" in figure 2) may be fixed, e.g. fixed per sensor. For example, a first sensor may have a time base of 30 minutes per time slot. In this example, the sensor will read an entry from the sampling pattern every 30 minutes. If that entry is a '0', no measurement will be taken. If the entry is a '1', the sensor will take a measurement of the physiological signal.

The time base for each time slot may be fixed across multiple sensors. For example, the time base for each time slot may be 1 minute for multiple sensors. The time base of each time slot may be set by parameters that are defined with the sampling pattern. The parameters may be set by the processor. The parameters may define other features of the sampling pattern such as the start time for the sampling pattern.

In another example, when the sampling pattern is expressed as a matrix as in figure 2b, each row of the sampling pattern may correspond to a different sensor. The sampling pattern may in this case have a fixed time base for each time slot for all the sensors. For example, the columns of the matrix may correspond to time base of 1 minute per time slot. The first row may correspond to one sensor, and the next row may correspond to a different sensor. As an example, if the first row of the matrix contained values [0, 1, 0, 1, 0, 1...] then the first sensor will take a reading every other minute - e.g. every other time slot, where the time slot is 1 minute. As another example, if the third row of the matrix contained values [1, 0, 0, 0, 1, 0, 0, 0...] then the third sensor will take a reading every 5 minutes. The values in each row do not need to repeat according to a pattern as in the above two examples. For example, they may be randomly distributed values.

In general, a sampling pattern may be any set of values suitable to indicate when a sensor is to take a measurement or sample. The set of values of a sampling pattern may be organised in any suitable manner. For example, the set of values may be a 1-dimensional string of values. In other examples, the set of values may be represented as a mathematical matrix of values.

The sampling patterns may be updated so as to adjust the frequency with which each sensor measures physiological signals. This means that the rate at which each sensor takes samples may vary, e.g. throughout the day. A sensor may receive a sampling pattern that means that the sensor does not take any measurements at all. This may remain the case until that sensor receives an updated sampling pattern. In this way, sensors may sample the respective physiological signal at varying rates over time, depending on how often each sensor receives an updated sampling pattern.

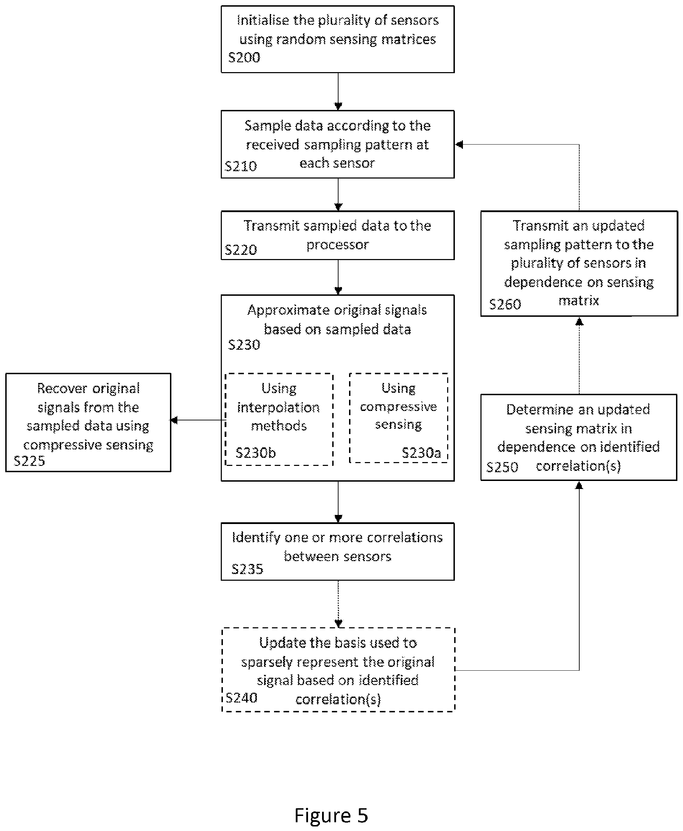

The sampling patterns may be updated periodically and/or in response to the processor determining that a sampling pattern is to be updated - e.g. in accordance with step S250 described below with reference to figure 5. The process of updating the sampling pattern relies on an analysis of the sampled data and will be described in more detail below with reference to compressive sensing. The processor 3 may determine when the sampling patterns are to be updated (e.g. how often the sampling patterns are to be updated). For example, the processor may periodically evaluate whether the sampling patterns need to be updated. For example, the processor may decide whether to update any of the sampling patterns every 6 hours. The processor may evaluate whether or not to update the sampling patterns according to an update cycle - that is, the update cycle may determine how often the processor is to consider updating the sampling patterns. For example, if the processor evaluates whether to update any of the sampling patterns every 5 minutes, the period of the update cycle is 5 minutes. The processor evaluates whether to update the sampling patterns by analysing the historical data of each sensor (e.g., the data measured by each sensor in previous time intervals). For example, if the variance of the data measured in the previous update cycle for a particular sensor is below a threshold, it means there has been no significant variation in the data measured by that sensor during that time period, so the sampling pattern for that sensor for the next update cycle does not need to be updated. Further detail on the process of updating the sampling pattern is discussed later.

Updated sampling patterns are transmitted to the plurality of sensors from the processor 3. As an example, if it is determined by the processor that a sensor no longer needs to sample data as frequently (for example), an updated sampling pattern will be transmitted to that sensor and the sensor will adapt the frequency of the sampling in dependence on the received sampling pattern. On the other hand, if it is determined by the processor that a sensor needs to sample the respective physiological signal more frequently, because, for example, the sampled data exhibits some unusual signs, then the processor 3 will send that sensor an updated sampling pattern that increases the sampling frequency of that sensor. The processor may decide that no change is needed for a sampling pattern, in which case the respective sensor will not receive an updated sampling pattern.

The plurality of sensors within the system 1 have limited processing power and limited memory. This is advantageous as it allows the sensors to be small, low power, and relatively low cost. The sensors have the capacity to receive a sampling pattern that determines when the sensor is to take a sample. If the sensors comprise a memory 23, the memory may store the sampling pattern. The sampling patterns are suitably binary patterns. That is, the sampling pattern may simply comprise 0s and 1s. In this case, the sensor is configured to take a sample on reading "1" and not to take a sample on reading "0" (or vice versa). For example, if the processor has determined that a sensor does not need to take any measurements, then that sensor will receive a sampling pattern containing only zeros. It is advantageous for the sampling patterns to be binary patterns as this reduces the complexity of processing the sampling pattern on each sensor. This allows the sensors to remain low-complexity, low-cost and low-power, meaning their battery life will last longer.

As described above, the plurality of sensors 2 sample data according to their respective sampling patterns. These sampling patterns may be updated throughout the day by the processor. The sensors 2 may be described as providing "continuous" monitoring of the user's physiological signals in that they are capable of continuous monitoring. This is because the sensors are designed to remain in place on the user allowing for continuous monitoring of the user's physiological signals. As will be appreciated by those skilled in the art, a "continuous" sensor may capture measurements according to any periodic or non-periodic sampling pattern; the use of the term "continuous" is to distinguish over measurements taken by sensors which are not continuously worn (e.g. temperature measurements manually taken using an oral thermometer).

The sensors 2 wirelessly transmit the data that they have sampled to the processor 3 (e.g. via the transceiver unit 21). The sampled data may be transmitted to the processor in real time - e.g. a data value may be transmitted in response to that data value being sampled. However, the process of transmitting data from the sensors to the processor is energy intensive and so it is advantageous if the frequency with which sampled data is transmitted can be reduced. Each sensor may wait until a certain amount of data has been sampled before transmitting the sampled data to the processor. For example, a sensor may transmit the sampled data once it has taken 5 samples. This is advantageous as the sensor will transmit data at a rate that is dependent on how many samples the sensor is taking. Alternatively, each sensor may wait a certain amount of time before transmitting the sampled data. For example, a sensor may transmit the sampled data periodically (e.g., every 1 minute).

How frequently each sensor transmits sampled data may be dependent on the sampling pattern received. For example, for a sensor that has received a sampling pattern of predominantly zeros (i.e., instructing the sensor to take very few measurements), there is no need for that sensor to transmit sampled data every 1 minute, for example, because the sensor may not have taken any measurements in that time. Instead, the sensor may transmit data every 10 minutes. Transmitting data from the sensors to the processor consumes a high proportion of the sensor's battery. As the sampling pattern varies for each sensor, the rate of transmission and/or the amount of data transmitted by each sensor will vary. Reducing the rate of transmission and/or the amount of data transmitted by each sensor increases the battery life of the sensors. In some examples, the sampling pattern may define when the sensor is to transmit its samples to the processor.

As described above, the processor 3 may transmit a respective sampling pattern to each sensor of the plurality of sensors. In some examples, the processor may transmit the sampling patterns for the sensors as one "master" sampling pattern. For example, the respective sampling pattern for each sensor may be transmitted within a single master matrix which comprises the respective sampling patterns for each sensor (e.g., where each row is a sampling pattern for a respective sensor). This may simplify the transmission from the processor to the sensors. In some examples, the processor may transmit a 'master' sampling pattern for groups of one or more sensors. For example, the processor may transmit a single master matrix for a subset of the plurality of sensors. The single master matrix may contain the respective sampling patterns for each sensor. In some examples, if there are one or more sensors that have the same sampling pattern (e.g. a group of similar sensors), then the processor may transmit one sampling pattern to the one or more sensors.

The processor 3 may comprise multiple elements.

The processor 3 shown in figure 1 comprises a receiving device 31 and a sensor controller 32. The sensor controller 32 may be any device with sufficient storage and processing capabilities. For example, the sensor controller 32 may be a smart-phone, a computer, or a cloud based server. The receiving device 31 and the sensor controller 32 may be in wired or wireless communication with each other.

The receiving device 31 sits between the sensor controller 32 and the plurality of sensors. Each sensor of the plurality of sensors transmits sampled data to the receiving device 31, e.g. wirelessly, such as using Bluetooth low energy (BLE). As shown in figure 1, the receiving device 31 may comprise a transceiver 311 for transmitting and receiving data. The receiving device then transmit the sampled data from the plurality of sensors to the sensor controller 32. The receiving device may compress the sampled data from the plurality of sensors prior to transmitting it to the sensor controller 32. In this case, the sensor controller 32 may need to decompress the received data from the receiving device 31 before processing the sampled data further.

The receiving device may be attachable to the user's body. For example, the receiving device may be a smart-watch. It may be advantageous to have the receiving device positioned on the user's wrist (or any other part of the body) so that the distance between the body sensors and the receiving device is a small as possible. Alternatively, the receiving device may be connected to the user via other means. The receiving device may be a pair of smart glasses, headgear, or any other form of wearable apparatus. The distance between the receiving device and the furthest sensor in the sensor network may be 1.5m or less. Keeping the distance between the sensors and the receiving device as small as possible means that transmission of the sampled data from the sensors to the receiving device is less energy consuming for the sensors. This improves the battery life of the sensors.

The receiving device may comprise a location tracker such as a GPS. In the case that the user's physiological signals are abnormal and dangerous, the receiving device may transmit the user's location to the emergency services, or to some other medical service.

When the processor comprises a receiving device 31 and a sensor controller 32, the receiving device 31 may transmit the sampling pattern(s) to the plurality of sensors. The receiving device may receive the respective sampling patterns for each sensor (or alternatively, a single 'master' sampling pattern for all of the sensors) from the sensor controller 32. Alternatively, the receiving device may determine the sampling pattern for each of the plurality of sensors, for example if the sensor controller 32 provides the necessary information needed to determine the sampling pattern to the receiving device 31. This will be explained in more detail below.

Each sampling pattern is derived from a sensing matrix. Details of how a sensing matrix is determined will now be discussed in the context of compressive sensing and sparse coding.

Compressive Sensing

Usually when taking samples of a signal, it is necessary to sample the signal at least twice the frequency of the maximum frequency of the signal being sampled in order to reproduce the original signal from the samples without a loss in information. This is called the Shannon/Nyquist sampling theorem. Sampling according to the Nyquist rate (i.e., twice the frequency of the maximum frequency of the signal being sampled) is energy intensive and results in a large amount of data being sampled, which inevitably must be compressed prior to its transmission.

Compressive sensing is a sampling technique that can recover an original signal (or a very good approximation of the original signal) through taking fewer samples than required by traditional sampling theory (i.e., the Shannon-Nyquist sampling theory). Compressive sensing relies on the fact that some signals can be described as "sparse". Often these signals are not naturally sparse (i.e., they are not sparse in their original basis) but become sparse once they are transformed into a different basis. For example, most natural images have sparse representations when they are transformed into the Fourier or Wavelet basis - in other words, when transformed into the Fourier or Wavelet basis, many of the image pixel coefficients will become zero or very small, and only a few of the pixels will have large coefficients.

A signal of length-N is called "K-sparse" if it can be written as a sum of K basis vectors, where K is significantly less than N.

Generally, any signal $x \in R^n$ can be expanded in a basis $\Psi = [\psi_1, \psi_2 ... \psi_n]$ so that it is expressed as:

$$x = \sum_{i=1}^{N} s_i \Psi_i \text{ or } x = \Psi s \qquad \text{(equation 1)}$$

where $s_i = \langle \mathbf{x}, \psi_i \rangle$. The signal **x** can equally be written as the basis matrix $\Psi$ multiplied by the coefficient vector **s.** The vectors **x** and **s** are equivalent representations of the same signal, with **x** in the time or space domain and **s** in the $\Psi$ domain. Representing the signal **x** in this way may also be equivalently described as transforming **x** with a "transform basis" $\Psi$ (e.g., the discrete cosine transform DCT).

The signal **x** is called *K-sparse* if it is a linear combination of only *K* basis vectors; that is, only *K* of the coefficients of $s_i$ in equation 1 are nonzero and (*N - K*) are zero. According to compressive sensing theory, the signal **x** is *compressible* if the representation of **x** in some basis as shown in equation 1 has just a few large coefficients and many small coefficients. In this case, the signal can be approximately written as a combination of the non-zero vectors, $x \sim \sum_{i=1}^{K} s_i \psi_i$ where K << N. In other words, a large amount of the signal can be discarded in the basis $\Psi$ without a large loss in information.

When a signal is K-sparse as described above, it is no longer necessary to take samples at twice the rate of the maximum frequency of the original signal. Instead, compressive sensing theory has shown that the original length-N signal **x** can be reconstructed from only M measurements, where K ≤ M < N, provided the original signal **x** is "K-sparse".

Taking samples of a signal can be thought of as multiplying the original signal by some matrix which results in the sampled data - i.e., a smaller length vector containing entries that are samples of the original signal. The matrix can be thought of as "pulling out" certain values of the original signal, the result being the sampled data. The matrix that multiplies with the original signal to produce the sampled data is called a measurement matrix, or equivalently, a sensing matrix (herein referred to as a sensing matrix).

The sampling patterns described with reference to figure 2 are derived from sensing matrices (e.g., each sampling pattern is derived from a respective sensing matrix). The sensing matrix represents the mathematical visualisation of how the signal is sampled. The sampling pattern is the information that is transmitted to the sensor and which instructs the sensor when to take a measurement of the respective physiological signal. As explained above, this may be a string of binary values, where a '1' indicates that the sensor is to take a sample, and a '0' indicates that the sensor does not take a sample. The sampling pattern may be expressed in matrix form. However, the sampling pattern when expressed in matrix form is not to be confused with the sensing matrix discussed below. The sampling pattern for each sensor is determined based on the sensing matrix for that signal. However, the sampling pattern and the sensing matrix are not necessarily the same. In some cases, the sensing matrix described below may be suitable for transmission to the sensor as a sampling pattern. In that case, the sensing matrix may be equivalent to the sampling pattern. However, it is to be understood that this is not necessarily the case.

Returning to compressive sensing, mathematically, the relationship between the sampled data for one physiological signal, the sensing matrix, and the original physiological signal can be written as:

$$\mathbf{y} = \mathbf{\Phi x} \qquad\qquad\qquad \text{(equation 2)}$$

Where $\mathbf{y}$ is the sampled data, $\mathbf{x}$ is the original signal, and $\Phi$ is the sensing matrix. The measurements $\mathbf{y}$ can be written as inner products between the signal $\mathbf{x}$ and elements of the sensing matrix $\Phi$, $y_j = \langle \mathbf{x}, \varphi_j \rangle$.

Compressive sensing theory determines a sensing matrix that can be used to reconstruct the original signal in its sparse form from as few samples as possible.

Equations 1 and 2 above can be combined. Consider a general linear measurement process that takes M measurements from the length-N signal $\mathbf{x}$ which is K-sparse in the domain $\Psi$. As described above, the measurements $\mathbf{y}$ can be written as inner products between the signal $\mathbf{x}$ and elements of the sensing matrix $\Phi$, $y_j = \langle \mathbf{x}, \varphi_j \rangle$. The original signal $\mathbf{x}$ can be written as a sum of basis vectors. Using the basis $\Psi$, by substituting $\mathbf{x} = \Psi\mathbf{s}$ from equation 1, $\mathbf{y}$ can be written as:

$$\mathbf{y} = \mathbf{\Phi x} = \mathbf{\Phi\,\Psi s} = \mathbf{Ds} \qquad\qquad\qquad \text{(equation 3)}$$

Where $y \in R^{1 \times M}$, $\Phi \in R^{M \times N}$, $\Psi \in R^{N \times N}$ and $s \in R^N$. $\mathbf{D}$ is an approximately orthogonal matrix.

Compressive sensing theory has shown that it is possible to recover the sparse representation $\mathbf{s}$ of the original signal $\mathbf{x}$ from only a few (M) samples of the signal (i.e., only from the measurements $\mathbf{y}$). Provided the basis $\Psi$ is known, the original signal $\mathbf{x}$ can be (approximately) reconstructed from the sparse signal $\mathbf{s}$.

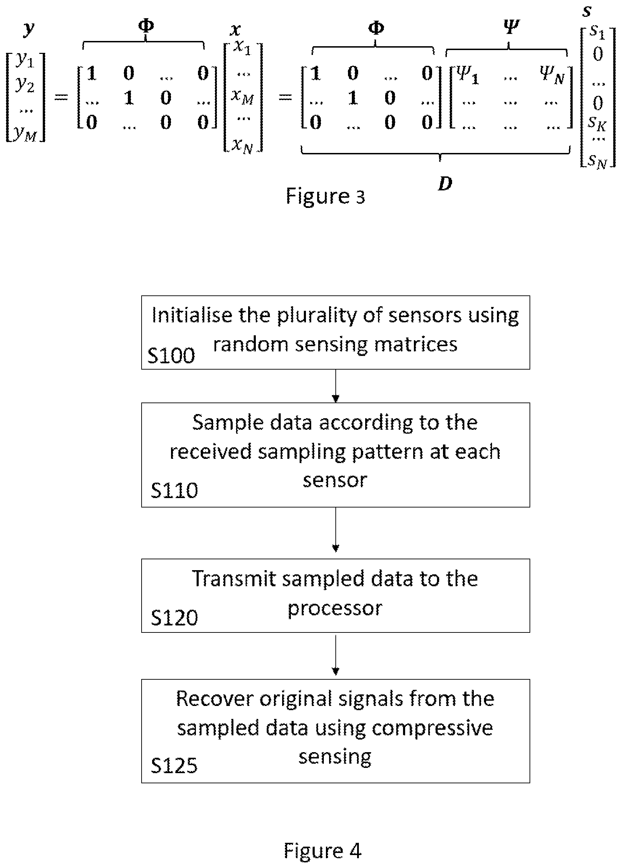

Figure 3 is an example mathematical visualisation of the relationship shown in equation 3. In figure 3, the matrix $\Phi$ is shown with only one "1" value per row, so that each y value corresponds to one x value. For example, $y_1 = x_1$. It may be the case that the matrix $\Phi$ contains more than one nonzero entry per row. For example, it could be the case that each y measurement is a combination of values of x. For example, $y_1 = 2x_1 + 3x_5$. For simplicity, herein we will treat each measurement of y as corresponding to one value of x.

The matrix $\Phi$ shown in figure 3 has M rows and N columns. In this way, the measurement vector $\mathbf{y}$ is not the same dimension as the discretised signal $\mathbf{x}$. In other words, the measurement vector $\mathbf{y}$ contains fewer values than $\mathbf{x}$, because $\mathbf{y}$ represents taking M samples of an N-length signal $\mathbf{x}$.

Various approaches may be taken to performing compressive sensing. Further details on the theory behind compressive sensing may be found in E. Candès, J. Romberg, and T. Tao, "Robust uncertainty principles: Exact signal reconstruction from highly incomplete frequency information," IEEE Trans. Inform. Theory, vol. 52, no. 2, pp. 489-509, Feb. 2006. See also D. Donoho, "Compressed sensing," IEEE Trans. Inform. Theory, vol. 52, no. 4, pp. 1289-1306, Apr. 2006.

Determining the sensing matrix

Compressive sensing techniques find the sensing matrix $\Phi$ that allows the approximate reconstruction of the length-N signal $\mathbf{x}$ from M < N measurements (the vector y). This relies on the idea of sparsity as described above, and the idea of "incoherence".

The main criterion in determining an appropriate sensing matrix $\Phi$ is that it should be as "incoherent" as possible with the sparsifying basis used to represent the original signal $\Psi$. Coherence is a measure of correlation between two vectors. Typically, the use of compressive sampling means that the rows $\{\varphi_j\}$ of $\Phi$ cannot sparsely represent the columns $\{\psi_i\}$ of $\Psi$ (and vice versa).

Mathematically, coherence is defined as

$$\mu\left(\Phi, \Psi\right) = \sqrt{n} \cdot \max_{1 \le k, j \le n} \left| \langle \varphi_k + \psi_j \rangle \right|$$

If the sensing matrix was completely incoherent with the sparsifying basis, then $\mu(\Phi, \Psi) \in [1, \sqrt{n}]$.

The processor 3 may determine the sensing matrix used to derive a sampling pattern for each sensor. If the processor 3 comprises a receiving device 31 and a sensor controller 32, the sensing matrices may be determined at the receiving device 31 and/or the sensor controller 32. A sampling pattern for each sensor may then be determined by the processor in dependence on the sensing matrix for the respective sensor.

Reconstructing the original signal

Each physiological signal may be reconstructed (equivalently referred to as "recovered") at the processor 3 using the above compressive sensing theory.

The signal reconstruction algorithm takes the M measurements in the vector **y**, the random measurement matrix $\Phi$ (or the random seed that generated it) and the basis $\Psi$ and reconstructs the length-N signal **x** or, equivalently, its sparse representation s.

For K-sparse signals, since M < N in equation 3, there are infinitely many vectors s' that satisfy **Ds'** = **y**. This is because if **Ds** = **y** then **D**(s + r) = y for any vector r in the null space N(**D**) of **D**. Therefore, the signal reconstruction algorithm aims to find the signal's sparse coefficient vector in the (N - M)-dimensional translated null space H = N (**D**) + s.

It has been shown (E Candes, 2006) that the sparse signal can be recovered using the "$l_1$- norm" reconstruction:

$$\hat{s} = argmin\|s'\|_1 \; subject \; to \; \boldsymbol{D}s' = \boldsymbol{y}$$

Where **D** = $\Phi$ $\Psi$.

The $l_1$- norm can recover K-sparse signals using only $M \geq cK \log\left(\frac{N}{K}\right)$ independently identically distributed Gaussian measurements. This is a convex optimization (i.e., not computationally hard) problem that conveniently reduces to a linear program known as basis pursuit whose computational complexity is about $O(N^3)$.

The original reconstructed signal **x** is found from the sparse signal s since **x** = $\Psi$**s.**

The $l_1$-norm minimization method above is not the only method used to recover the sparse signal s. Other methods such as via the $l_0$-norm method, orthogonal matching pursuit (OMP), iterative hard thresholding (IHT) or other greedy algorithms may be used.

Using random sensing matrices with a fixed basis

It has been found that random sensing matrices are mostly incoherent with *any* fixed basis. That is, a random sensing matrix has been found to be largely incoherent with well-known basis functions such as the Fourier basis, Wavelet basis, or Discrete cosine transformation (DCT). Furthermore, it has been found that sensing matrices with independently identically distributed (i.i.d) entries such as a Gaussian distribution also show low coherence with these fixed bases or transforms.

The body sensor system 1 (e.g. a wireless body sensor system) of the present application may use a random sampling pattern, derived from a random sensing matrix, with which to sample each physiological signal (i.e., use a random sensing matrix with which to determine the sampling pattern for each sensor). In other words, each of the plurality of sensors 2 in the wireless body sensor network may receive a random sampling pattern. In some examples, each sensor may be already initialised with a random sampling pattern (i.e. each sensor may not need to receive a random sampling pattern prior to starting to sample data). Each sensor may take random samples of the respective physiological signal. When it comes to recovering each respective physiological signal at the processor, the processor may use any suitable fixed basis with which to represent each physiological signal. Typically, any basis will be largely incoherent with a random sensing matrix, and any signal is likely to be somewhat sparse when represented in a suitable basis. For example, each physiological signal may be sparse when Fourier transformed to a Fourier basis. The processor can recover the (approximate) original physiological signal from the sampled data, the random sensing matrix used to obtain said sampled data from the sampling pattern, and the fixed basis that is being used to sparsely represent the original signal (e.g. the Fourier basis).

Figure 4 shows an example method for sampling according to random sensing matrices and a fixed basis. Step S125 of recovering the original signal (e.g. each physiological signal) may be found with the $l_1$-norm minimization method described above, or by any alternative method as is known in the field of compressive sensing.

The above described approach allows reconstruction of the sampled signals by taking fewer samples than required by the Shannon-Nyquist sampling theorem. A system as described above that uses random sampling patterns for the body sensors derived from random sensing matrices and that reconstructs the physiological signals by using a fixed basis such as the Fourier basis has the advantage that it is simple to implement and is effective at reducing the number of samples needed to reconstruct the original signals.

Correlation between sensors

The inventors have appreciated that there is likely to be correlation between some physiological signals. For example, it is likely that when a user's heart rate is elevated, their respiration rate and their temperature may also be elevated. When there is a strong correlation between two physiological signals (for example), it may be unnecessary to sample both signals as frequently. This is because sampling one of the signals may provide enough information to also monitor the related physiological signal. On the other hand, if the sampled data relating to one physiological signal appears abnormal compared with historic data, it may be advantageous to not only sample the abnormal physiological signal more frequently, but to also increase the frequency of sampling of a related physiological signal. As another example, if the sampled data from two sensors suggests very poor correlation between two respective physiological signals sampled by the two sensors, when it is expected that those two physiological signals should exhibit a strong correlation (e.g., heart rate and respiration rate), the sampling rate for both sensors may be increased. Additionally, or alternatively, a third sensor may increase its sampling rate or awake from sleep mode to sample a related physiological signal (or the same physiological signal). By comparing the data sampled from all three sensors, it may be determined if the data from one sensor is likely to be incorrect or less reliable than the data obtained from the other two sensors at that time. This might suggest, for example, that a sensor has become misplaced on the user's body.

So, the physiological signals should ideally be analysed together to get the most out of the sampled data from each sensor.

Figure 5 shows an improved sampling method that takes into account relationships between the plurality of sensors.

At step S200, each sensor of the plurality of sensors starts with a random sampling pattern, as in the previous example. This may be derived from a Gaussian sensing matrix or a random binary sensing matrix. The sampling patterns may have random entries according to the Bernoulli distribution, for example. The random sampling patterns may be transmitted to the plurality of sensors from the processor (or alternatively the sensors may already be initialised with a random sampling pattern). The sensing matrix used to derive the sampling patterns in the first iteration of the method (if required) is denoted $\Phi_{t0}$ (e.g at t=0).

At step S210, each physiological signal is sampled according to the received sampling pattern defined by the random sensing matrix (or according to the sampling pattern initially present in the sensors). As explained above, for a binary sampling pattern, a '1' may indicate to the sensor that it takes a measurement, and a '0' may indicate that the sensor is to remain idle. Each physiological signal is sampled according to the sampling pattern for the respective sensor. Samples are taken in this way until the respective sensors receive an updated sampling pattern. The data sampled during the first iteration of the method may be represented as $\mathbf{y}_{t0}$.

At step S220, the sampled data is transmitted (e.g., from the plurality of sensors to the processor). If the processor comprises a receiving device and a sensor controller, each sensor transmits sampled data to the receiving device. The sensors may transmit the sampled data via the transceiver 22. If the processor comprises a receiving device, the receiving device may collate the data from each sensor. The receiving device compresses the sampled data from the plurality of sensors. The receiving device then transmits the compressed sampled data from the plurality of sensors to the sensor controller part of the processor (e.g., to the cloud).

The processor receives the sampled data. If the sampled data has been compressed (e.g., by the receiving device) prior to transmission, then the processor first decompresses the received data. If the processor comprises a receiving device and a sensor controller, the sensor controller may decompress the sampled data received from the receiving device.

At this point, the processor has sufficient information to reconstruct each original physiological signals from the sampled data for that signal, the random sensing matrix used to sample that signal, and a fixed basis such as a Fourier basis. The processor may then go on to consider correlations between reconstructed physiological signals.

However, the inventors have appreciated that this reconstruction step is not necessarily required in order to analyse the sampled data for correlations and to update the sensing matrices.

Figure 5 shows two different methods that may be used at step S230 to approximate each physiological signal, before proceeding to determine correlation between signals.

Step S230a reconstructs the original physiological signals using the compressive sensing techniques described above, such as with the orthogonal matching pursuit algorithm. The processor uses the sampled data corresponding to the physiological signal, the sensing matrix used to derive the sampling pattern for the physiological signal, and a basis that is incoherent with sensing matrix, to recover each physiological signal. Initially, at time t=0 during the first iteration of the method, the sensing matrix is random and so the basis used to sparsely represent each physiological signal may be any suitable basis such as the Fourier basis. This is because any suitable basis is largely incoherent with a random sensing matrix. As the method repeats itself over several iterations, the processor receives more sampled data from each sensor, and the sensing matrix and the basis that is used to sparsely represent each physiological signal may be updated, as will be explained later on with reference to step S240.

Alternatively or additionally, for the purposes of adapting the sampling pattern on each sensor, the physiological signals may be approximated without using the compressive sensing reconstruction technique performed in step S230a. At step S230b, each physiological signal is approximated from the sampled data using approximation methods as are already known in the art. For example, the sampled data may be used to approximate the original signal using known estimation techniques, such as interpolation. This is a less computationally intensive procedure than reconstructing the original physiological signals using compressive sensing, and so can be performed at devices with lower computational power. For example, step S230b may be performed on the processor without needing to connect to the cloud. This has the advantage that step S230b can be performed more quickly on the processor, or even on the sensors, and so the response time for the sampling patterns to be updated can be increased.

Full reconstruction of the original physiological signals using compressive sensing techniques as described above is performed in addition to approximating the physiological signals using step S230b. This is shown schematically in figure 5 by the arrow going from step S230b to step S225. If step S230b is taken instead of S230a, the original signals are still recovered using compressive sensing techniques. However, it has been determined that this step is not required in order to analyse correlation and adapt the sensing matrices in real-time (as step S230b may be used instead). Therefore, the reconstruction of the sampled data may occur at any time after the sampled data has been transmitted to the processor. For example, the processor may be configured to display the reconstructed signals (as reconstructed via compressive sensing techniques) to the user a certain amount of time after the sensors have taken their measurements. Delaying the full reconstruction of the original signals in this way allows the processor to focus on adapting the sensing matrix in real-time using step S230b. The reconstruction of the original signals using compressive sensing provides a more accurate picture of the history of the signal, so reconstructing the signal with compressive sensing is still required for the system to thoroughly monitor the user's physiological data. The reconstructed signals may be displayed to the user of the body sensors on a smart device, for example, and may be available to view a certain amount of time after the samples have been taken. However, for the adaptation of the sampling frequency on the sensors, step 230b may provide a quicker and simpler approach to reactively changing the sensor sampling patterns. The reconstruction of the signals in step S230a may be used to verify that the estimates of each physiological signal in step S230b are the same or similar to the reconstructed physiological signals determined with more intensive compressive sensing techniques, to within a certain degree.

Examples of approximation techniques for estimating each physiological signal from the sampled measurements that may be used in step S230b will now be described.

In one example, each physiological signal may be approximated from the samples using interpolation techniques. An interpolated version of the physiological signal measured by each sensor can be estimated using linear interpolation, polynomial interpolation, or spline techniques, for example.

As another example, principal component analysis (PCA) may be used to estimate each physiological signal from the under sampled measurements. As is known to the skilled person, PCA can be used to "fill in the gaps" in an under sampled signal to form an estimate of the complete signal. For example, in PCA, the sampled measurements are transformed into a different vector space represented by eigenvectors and eigenvalues. In performing this transformation, parts of the missing signal are represented in the new space such that when the measurements are transformed back into their original vector space, the missing values in the samples have been approximately filled in.

Other suitable approximation methods may be used to determine an estimate of the original physiological signal from the measurements taken at the sensor(s).

As mentioned above, performing step S230b may be less computationally intensive than performing step S230a, making it more suitable for being performed more quickly on the processor. When using the approximated signals as in step S230b, it may be desirable to periodically or occasionally perform step S230a, in addition to step S230b, to compare the results of the reconstructed and approximated physiological signals.

It is to be understood that both step S230a and step S230b form an approximation of the original physiological signals from the sampled data. However, reconstruction using compressive sensing techniques (as used in step S230a) is likely to be more accurate than interpolation approximation methods (as used in step S230b). For the sake of distinguishing the approximated physiological signals obtained via the two techniques, a "reconstructed" physiological signal herein refers to a physiological signal as reconstructed using compressive sensing techniques (as in step S230a), and an "interpolated" physiological signal herein refers to a physiological signal as approximated not using compressive sensing techniques (as in step S230b). As mentioned above, "interpolation" is just an example of an estimation technique that can be used in step 230b and is not intended to limit step 230b to only using interpolation.

Once the physiological signals have been approximated (e.g., reconstructed as in step 230a, or interpolated as in step 230b) correlation in the approximated physiological signals can be analysed.

Correlation analysis may involve a comparison between different physiological signals, or a comparison of the same physiological signal but sampled from different sensors. Correlation analysis refers to any statistical technique that can be used to indicate to what degree two or more variables are related. Correlation between two or more sensors or physiological signals may not be linear. Correlation may be inverse, in that one physiological signal shows an increased amplitude and/or frequency and the other physiological signal shows a corresponding decrease in amplitude and/or frequency.

The comparison between signals may be performed by determining a measure of correlation between two or more physiological signals. A measure of correlation between two or more physiological signals may be used to determine whether to update the sensing matrices for one or more sensors, and by how much. The sensing matrices that are updated may not be the sensing matrices corresponding to the two or more physiological signals for which the measure of correlation was detected.

The rate of change of the measure of correlation (i.e., how much the measure of correlation deviates from its previous value) may be used to determine whether to update one or more sensing matrices. The rate of change of the measure of correlation may be monitored depending on previously detected measures of correlation (e.g., an average measure of correlation). The deviation in the measure of correlation (e.g., the rate of change) may be determined based on an expected measure of correlation. The expected measure of correlation may be predefined, or may adapt depending on a rolling average of the measure of correlation for the two or more physiological signals. The expected measure of correlation may be equivalent to the measure of correlation determined for the two or more physiological signals previously.

As an example, if two physiological signals usually display an expected measure of correlation (for example, a correlation coefficient close to 1), a change in the measure of correlation is likely to indicate a change in the underlying physiological signals which may result in the sampling pattern(s) of the sensor(s) being updated.

The sensing matrices that are updated based on the measure of correlation between two or more physiological signals are not necessarily the sensing matrices that are used to sample the two or more physiological signals. For example, based on an identified correlation (or lack of correlation) between two physiological signals, a sensing matrix corresponding to a different physiological signal may be updated so as to sample more frequently.

As an example of a measure of correlation, correlation between signals can be determined using a regression line. The relationship between two approximated physiological signals (as determined in either step 230a or 230b) can be represented based on regression equation. A regression line can be calculated on a scatter diagram to illustrate change of one signal with respect to the other. For two physiological signals $x_1$ and $x_2$, the relationship between correlation and regression can be defined as:

$$r = m \frac{S_{x_1}}{S_{x_2}}$$

Where m denotes the slope of the linear regression line fitted to the scatter plot. $S_{x_1}$ and $S_{x_2}$ stands for the standard deviation of the signals $x_1$ and $x_2$, respectively. As mentioned above, the signals $x_1$ and $x_2$ may be the signals as reconstructed using compressive sensing, as determined in step 230a, or they may be the signals as approximated without compressive sensing, as determined in step 230b.

As another example, Pearson's correlation coefficient can be obtained as a measure of correlation between two physiological signals $x_1$ and $x_2$ via the equation:

$$\rho = \frac{cov\,(x_1, x_2)}{S_{x_1} S_{x_2}}$$

Where $cov\,(x_1, x_2)$ is the covariance matrix between the two physiological signals. A value of $\rho = 0$ implies the independence of the signals $x_1$ and $x_2$, meaning there is no correlation. Conversely, as $\rho$ approaches 1 or -1, the correlation between the two signals increases. A correlation coefficient of -1 indicates a perfect inverse linear relationship, and a correlation coefficient of 1 indicates a perfect increasing linear correlation. Hence, this can be a measure of degree of correlation.

Any other suitable techniques for determining correlation between two signals may be used. For example, Spearman's rank correlation coefficient may be used as a measure of correlation. Multiple techniques may be used in combination.

The examples described above apply to two physiological signals, however the above concepts can be extended to more than two signals. When obtaining the correlation between more than two signals, a correlation matrix will be formed.

As an example of two correlated sensors, a photoplethysmography (PPG) sensor and an electrocardiogram (ECG) sensor both measure heart rate. They are therefore likely to be highly correlated. When abnormalities or changes in the heart rate are detected in one sensor, the processor may increase the sampling rate for both sensors. A PPG sensor is a simpler and less energy intensive way of monitoring heart rate than with an ECG sensor. For this reason, the PPG sensor may be used more frequently than the ECG. To save power, the ECG sensor may be switched off in normal use. However, an ECG sensor is likely to be more accurate than a PPG sensor. In cases where there is an increased change in the heart rate data from the PPG sensor, the processor may turn on the ECG sensor to start sampling via the ECG sensor, and/or increase the sampling rate on the ECG sensor. The ECG sensor may only be used occasionally, for example when the PPG sensor data displays anomalies, so that power is saved on the sensors.

As an example, when both the ECG and PPG sensors are in use, it is expected that the data sampled by both sensors (relating to the user's heart rate) should be highly correlated. If this is not the case, the sampling rate on both sensors may be increased. If the data from both sensors continues to show lack of correlation even when the sampling rate on both sensors is increased, the processor may compare the heart rate data to another related physiological signal from a third sensor. Through comparing the correlation between sensors that measure the same physiological signal, and sensors that measure a related physiological signal which is expected to correlate with the first physiological signal, it can be determined from a comparison of the data from three or more sensors as to which physiological signal is most accurate, and if there are any faults with a particular sensor.

As another example, it may be possible to infer blood pressure from PPG and ECG sensors. An ECG sensor measures the electrical activity of the heart, which provides information about the heart rate and rhythm. Monitoring the heart rate and rhythm is crucial for users who suffer from heart arrhythmia. Furthermore, simultaneous PPG and ECG measurements may be used to infer blood pressure of the user, which is another important metric in assessing the health of the user.

Once correlations have been detected among physiological signals, it can be determined at step S250 whether or not some parts of each physiological signal may be ignored. For example, if the user's heart rate and respiration rate are both within an expected range (i.e. within an average range calculated from historical data) and are highly correlated, it may be determined that the user's respiration rate is not conveying useful information that is not already learnt from the heart rate. In that case, parts of the respiration rate signal may be ignored or effectively deleted. This increases the sparsity of the respiration rate signal and hence lowers the number of samples needed to accurately represent it. In this case, an updated sensing matrix for each sensor is determined that reduces the frequency of samples taken at each sensor.

The processor may assign each physiological signal a different level of priority (i.e., importance). For example, heart rate may be the highest priority physiological signal for a particular user. This means that when the processor determines which physiological signals can be sampled less frequently, the processor will not choose to reduce the sampling frequency of the sensor that samples heart rate. Instead, the processor may reduce the sampling frequency of a correlated physiological signal that has been assigned a lower priority level or lower importance. The priority of each physiological signal may vary over time, depending on historic data. Machine learning algorithms at the processor may build up a picture of user specific priorities. These may vary depending on time of day. Physiological signals that are sampled at the same position on the user (e.g., the wrist) may exhibit high levels of correlation due to a systematic error. When analysing the physiological signals to detect correlation, the processor may take into account the position on the body of the respective sensor. As mentioned above, correlations between three or more sensors may be used to determine if the data from one sensor is inaccurate.

At step S250, updated sensing matrices are determined in dependence on the one or more identified correlations. The measure of correlation identified at step S235 may be used to determine whether to update the sensing matrices, and by how much. For example, if the measure of correlation between two or more physiological signals exceeds a threshold value or absolute value, it may be determined that the two or more physiological signals are strongly correlated such that the sampling frequency can be reduced on some or all of the physiological signals. In another example, if the measure of correlation between two or more physiological signals dips below a predefined threshold, it may be determined that the two or more physiological signals need to be sampled more frequently, as one or more of the physiological signals may be exhibiting abnormal behaviour. As mentioned above, step S250 may include determining a change in the measure of correlation between signals compared with the historical measure of correlation between those signals. A particularly sharp change in the measure of correlation may indicate that one or more of the physiological signals may be exhibiting abnormal behaviour and the sampling frequency of the one or more physiological signals may need to be increased. The change in the measure of correlation may be compared against a threshold value (e.g. representing an acceptable amount of deviation), the sensing matrices being updated in response to the change in the correlation exceeding that threshold value.

As mentioned above, updating the sensing matrices in dependence on the one or more identified correlations may involve updating sampling patterns for two or more sensors that have been determined to be highly correlated such that at least one of the sensors has a reduced sampling frequency. To determine which sensor has a reduced sampling frequency, the processor may reduce the sampling frequency of the lower priority physiological signal. Alternatively, for two sensors that have been determined to only weakly correlate, their respective sampling patterns may be updated such that the two sensors sample at alternate times. That is, whilst one sensor is not taking a measurement, the other sensor is taking a measurement, and vice versa. This ensures that information is not lost between each sensor taking a sample. When the processor considers the sampling patterns for correlated physiological signals together as described above, this may be described as updating the two sampling patterns interdependently.

Correlations have been discussed above with reference to pairs of physiological signals, however it is to be understood that the techniques described herein may apply to any number of physiological signals.

At step S260, updated sampling patterns, derived from the updated sensing matrices, are transmitted to the plurality of sensors. The updated sampling patterns will replace the random sampling patterns initially used to sample the first set of data and the process will repeat from step S210. It may be the case that the processor does not need to update every sampling pattern for each respective sensor. For example, the processor may only update one or two of the sampling patterns out of the plurality of sampling patterns for each respective sensor.

The first iteration of the method shown in figure 5 includes the first step S200 of initialising the plurality of sensors using random sampling patterns. Once steps S200 to S260 have been performed once by the system 1, the sampling patterns are no longer necessarily random, as they may have been replaced by updated sampling patterns at step S260 of the previous iteration. For a processor that evaluates the sampling patterns periodically according to an update cycle, the steps S210 to S260 of figure 5 are repeated every update cycle (e.g., every 5 minutes).

In addition to the steps described above, step S240 may optionally be included. Step S240 is be described in more detail below.

Updating the transform basis

Using a fixed transform basis to sparsely represent the original signal is convenient because it is simple to implement and it works well in most cases, particularly for compressing natural images. However, it may be advantageous to adapt the basis in which each physiological signal is represented to further reduce the number of samples taken to recreate each physiological signal. Furthermore, the process of adapting the basis in which each physiological signal is represented can be used to monitor changes in the physiological signal itself, which can be used to detect whether any abnormality in the signal is present. This will be explained in more detail below.

There is a branch of machine learning called sparse coding which aims to find a "dictionary" that most sparsely represents a set of input data. A dictionary is similar to a basis, however it may be "overcomplete". In other words, a dictionary is a basis matrix whose elements may not all be used in representing the original signal. The columns of a dictionary are also called atoms. The columns (or atoms) represent the data. Each column is approximately orthogonal. In other words, each column is approximately independent.

Given a set of "training data", several algorithms (e.g., K-SVD or LASSO) have been developed that find a dictionary that can be used to represent that training data in the sparsest way.

Mathematically, these algorithms determine a dictionary D for a sparse representation **s** of a signal **x**, given some training data $\{x_i\}^{i=K}$, where the training data can be written as:

$$\{x_i\}^{i=K} = \mathbf{Ds}$$

The dictionary may be defined by the following minimization problem:

$$\min_{\mathbf{D},\ \mathbf{s}_i} \sum_{i=1}^{K} \|\mathbf{x}_i - \mathbf{D}\mathbf{s}_i\|_2^2 + \lambda\|\mathbf{s}_i\|_1$$

The K-SVD algorithm, as an example of a minimization algorithm, can find a dictionary D from the training samples $\{x_i\}^{i=K}$ that results in the sparsest representation **s.**

The inventors have realised that sparse coding may be used with training data from the plurality of sensors in the wireless body sensor network to represent the physiological signals in their sparsest, or a sparser, form, as opposed to when a fixed basis such as a Fourier basis is used to sparsely represent the signals. The sparser the representation of the physiological signal, the fewer samples need to be taken to accurately reconstruct the signal at the processor. So, it is advantageous if the physiological signals can be represented in a basis (or equivalently over complete dictionary) that results in the sparsest representation **s.** Furthermore, by iteratively adapting the basis (or equivalently overcomplete dictionary) used to sparsely represent each physiological signal over time, any changes to the basis can be used to indicate changes in the underlying physiological signal. For example, the basis used to sparsely represent a very regular physiological signal will not change much over time, even as more and more samples of that physiological signal are taken. With a very regular signal (e.g., one of a nearly constant frequency and amplitude), updates to the sparse basis will be small. However, if, for some reason, the regular signal suddenly becomes irregular, the basis used to sparsely represent the signal will no longer accurately represent the irregular signal. In the next iteration, there will be significant changes to the basis in order to try to sparsely represent the new irregular signal. Such changes to the sparse basis may flag an abnormal condition in the physiological signal. Thus, changes in the physiological signal are reflected in the amount by which the sparse basis used to represent that signal changes over time.

A further method of adaptively capturing physiological signals will now be described, with reference to step S240 in figure 5. Step S240 is an optional addition to the method shown in figure 5 which can improve the sampling technique of the sensors by further reducing the number of samples needed to accurately reconstruct the data. Step S240 also provides an additional metric with which to monitor changes to the underlying physiological signals. The method as described below includes the step S240.

As explained above, at step S230, each physiological signal is approximated from the sampled data. This may be either through reconstructed using compressive sensing, or through interpolation techniques as explained above with reference to S230a and S230b. At step S235, one or more correlations may be detected between the approximated physiological signals, as described above.

Once each physiological signal has been approximated from the sampled data (e.g., in either step S230a or step S230b) and correlations between sensors have been accounted for at step S235, the processor then employs sparse coding algorithms such as K-SVD to find a basis $\Psi_{t_1}$ that represents each approximated physiological signal with the sparsest, or a sparser, vector. This process is performed at step S240. The recovered or interpolated physiological signal acts as the "training samples" $\{x_i\}$ for the sparse coding algorithms such as K-SVD or LASSO. The sparse coding algorithms then determine a basis from the training samples (i.e., the approximated physiological data) that results in a sparser representation of that data.

Taking the algorithm K-SVD as an example, the basis $\Psi_{t_1}$ is evolved or updated through performing a minimization algorithm as explained above. The correlation between the physiological signals may be used as an additional constraint in this minimization problem, such that the evolved basis $\Psi_{t_1}$ takes into account the correlation between sensors. This enables the basis to find a sparser representation of each physiological signal such that fewer samples can be taken of the signal, thus saving power on the sensors.

Once a basis has been determined for each physiological signal, the processor then updates the respective sensing matrix for that signal. This is performed using machine learning algorithms that find the sensing matrix $\Phi$ that is most incoherent (or more incoherent than before) with the basis $\Psi_{t_1}$, as explained above in the section titled "Determining the sensing matrix". This step of finding an updated sensing matrix that is incoherent with the updated basis is not shown explicitly in figure 5 but is included in the step S250 when the previous step S240 has occurred.

The updated sensing matrices $\Phi_{t_1}$ are used to determine updated sampling patterns for the respective sensors. The updated sampling patterns are transmitted to the sensors at step S260 and the process repeats from step S210. If a processor is updating the sampling matrices periodically (i.e., every 5 minutes), steps S210 to S260 last the period of an update cycle.

Each time the process shown in figure 5 repeats (including the step S240), the basis $\Psi_t$ for each signal is evolved or adapted slightly. As the system gathers more samples, each basis can be fine-tuned to more sparsely represent each physiological signal, and the sensing matrices are correspondingly altered.

When the steps S210-S260 are repeated following the first iteration of the method steps S200 to S260, the sensing matrix for each sensor may no longer be random because it may have been replaced at S260 with an updated sensing matrix from the previous iteration. When including step S240 in the method shown in figure 5, the updated sensing matrices are determined to be incoherent with the basis $\Psi_{t_1}$ that was determined by the processor using the approximated physiological signal in the previous iteration. Furthermore, after the first iteration of steps S200-S260, the bases used to sparsely represent each physiological signal may no longer be a fixed basis such as the Fourier basis. Instead, the basis used to sparsely represent each signal will be the basis as updated in step S240, which is the basis $\Psi_{t_1}$. When the processor reconstructs each physiological signal from the sampled data in a succeeding iteration, e.g., at step S225, the processor may use the updated determined basis $\Psi_{t_1}$ from the previous iteration of the method to recover the sparse signal **s,** when the method shown in figure 5 includes step S240.

It may be that some physiological signals cannot be more sparsely represented with an updated basis, or that there is no need to update the sampling pattern for that sensor (e.g. during a particular update cycle). The processor may not update the sensing matrix used to determine the sampling pattern for every sensor when performing step S250.

The amount by which the sparse basis $\Psi_t$ changes in each iteration can be used as a metric to determine how much the corresponding physiological signal being represented by the sparse basis is changing. Usually, the basis $\Psi_{t_1}$ will vary slightly with each iteration, but provided the physiological signal being measured is fairly consistent (e.g., a consistent amplitude and frequency), any changes to the basis $\Psi_t$ will be small. If the changes in the updated basis $\Psi_t$ exceed a threshold value, it may be determined that the physiological signal is behaving abnormally. This may be an indication of the user having a heart attack, for example.

Changes to the basis $\Psi_t$ may be quantified in different ways. For example, the difference between the basis $\Psi_{t_1}$ (i.e., at time t=1) and the basis at $\Psi_{t_2}$ (i.e., at time t=2) may be used as an indication of the change. If this difference is above a threshold value, the processor may respond with an alarm and an increased sampling rate, for example.

As another example, since the basis $\Psi_t$ can be determined via a minimization problem (e.g., in the K-SVD

algorithm), the basis $\Psi_{t_1}$ that is determined to be the sparsest representation of the training data $\{\boldsymbol{x}_{t_1}\}$ will have a certain amount of acceptable error in the minimization algorithm. When applying an updated basis $\Psi_{t_2}$ to the same training data, the minimization algorithm is likely to produce a different amount of error. If the difference in error is above a threshold value, the processor may respond accordingly.

The above described method may provide a system for adaptively capturing data that requires the sensors to take a reduced number of samples. This is because when sampled data is recovered at the processor, the processor may determine a sparser representation of each physiological signal in an updated basis. The more sparse the representation of each physiological signal, the lower the number of samples needed to be able to reproduce that signal at the processor. Taking fewer samples at each sensor increases the battery life of each sensor in two ways - the first is that each sensor can take fewer measurements which takes up less energy, but also each sensor transmits a lower amount of data to the processor. The latter is the more energy consuming task.

The sampled data is likely to contain a large amount of noise. For example, the sensors are likely to be affected by the user's daily activities such as exercise or a change in the surrounding conditions (i.e., when the user is outdoors). Any muscle movement may cause artifacts in the data. As a result, the sparsity of the acquired signals may significantly change over time. Removing noise before compression is not possible as it greatly increases the circuitry complexity of each sensor, and conflicts with the energy constraints.

In the proposed system and the above described methods, the basis and sampling matrices are adjusted over time, so the noise in the data can be frequently adjusted for. In order to prolong the battery life of the sensors and keep their circuitry as simple as possible, artifact removal and preprocessing are applied at the processor where the energy consumption is not a concern.

In addition, one or more of the sensors may detect variables that are likely to cause noise in the other sensors. For example, the system may include a physiological sensor to measure movement - e.g., an accelerometer. As another example, the system may include a physiological sensor to measure the ambient temperature (e.g., the temperature of the user's surroundings). The processor may compare the sampled data from such sensors to identify correlations between these sensors and the remaining sensors in the system so as to correct for errors caused by external factors. For example, the processor may detect a correlation between the sensor measuring movement and another sensor. Where a strong correlation is present, the processor may determine that one of the sensors is experiencing noise from the user moving around, for example. As another example, if there is a strong correlation between the ambient temperature measured, and the user's temperature, then the processor can determine that the user is not experiencing a high temperature but is instead being affected by the weather. On the other hand, a lack of correlation between the two sensors may indicate that the user has an usually high temperature compared with the surrounding air temperature.

The applicant hereby discloses in isolation each individual feature described herein and any combination of two or more such features, to the extent that such features or combinations are capable of being carried out based on the present specification as a whole in the light of the common general knowledge of a person skilled in the art, irrespective of whether such features or combinations of features solve any problems disclosed herein, and without limitation to the scope of the claims. The applicant indicates that aspects of the present invention may consist of any such individual feature or combination of features. In view of the foregoing description, it will be evident to a person skilled in the art that various modifications may be made within the scope of the invention.

**Claims**

1. A system (1) for adaptively capturing physiological data using compressive sensing, the system comprising:

   a plurality of sensors (2) each configured to sample a respective physiological signal according to a sampling pattern (5) defined by a respective sensing matrix; and
   a processor (3) configured to:

   receive sampled data from the plurality of sensors (2);
   approximate the physiological signals from the sampled data;
   identify one or more correlations between the approximated physiological signals;
   update one or more of the sensing matrices in dependence on the one or more detected correlations; and
   transmit updated one or more sampling patterns (5) defined by the updated sensing matrices to the respective sensors of the plurality of sensors (2) for use in sampling the respective physiological signals.

2. The system as claimed in claim 1, wherein the processor is configured to approximate each physiological signal from the sampled data by, using the respective sensing matrix, solving for a sparse representation of the physiological signal by applying compressive sensing to the respective sampled data.

3. The system as claimed in any preceding claim, wherein the processor is configured to approximate each physiological signal from the sampled data using interpolation or principal component analysis.

4. The system as claimed in any preceding claim, wherein the processor is configured to update each sensing matrix by determining a respective basis that sparsely represents the respective approximated signal and determine a respective updated sensing matrix that is incoherent with the determined basis.

5. The system as claimed in claim 4 when dependent on claim 2, wherein solving for a sparse representation of each physiological signal is performed using the respective basis previously determined to sparsely represent the previously approximated physiological signal.

6. The system as claimed in claim 4 or 5, wherein the processor is configured to infer changes to the respective physiological signal based on a measure of change of the respective basis each time the respective sensing matrix is updated.

7. The system as claimed in any of claims 4 to 6, wherein, for each physiological signal, the respective basis is determined using a sparse coding algorithm that finds a sparser basis with which to represent the respective approximated signal, wherein the approximated signal is used as training data for the sparse coding algorithm.

8. The system as claimed in claim 7, wherein the identified correlations are used as an additional constraint in the sparse coding algorithm.

9. The system as claimed in any preceding claim when dependent on claim 2, wherein compressive sensing is applied using an $l_1$-norm so as to solve for the sparse representation of the physiological signal.

10. The system as claimed in any preceding claim, wherein the processor identifies a measure of correlation between two or more physiological signals.

11. The system as claimed in claim 10, wherein the processor updates one or more sensing matrices in dependence on whether the measure of correlation between two or more physiological signals deviates from an expected value by more than a predefined threshold, or in dependence on whether the rate of change of the measure of correlation between two or more physiological signals deviates from an expected value by more than a predefined threshold.

12. The system of any preceding claim, wherein the physiological signals are sparse signals such that each physiological signal of length N can be represented as a linear combination of K basis vectors, wherein K << N.

13. A method for adaptively capturing physiological data using compressive sensing, the method comprising:

sampling (S210) physiological signals at a plurality of sensors, each sensor being configured to sample a respective physiological signal according to a respective sampling pattern defined by a respective sensing matrix; and
approximating (S230) the physiological signals from data sampled by the sensors;
identifying (S235) one or more correlations between the approximated physiological signals;
updating (S250) one or more of the sensing matrices in dependence on the one or more identified correlations; and
transmitting (S260) updated one or more sampling patterns defined by the respective updated sensing matrices to respective sensors for use in sampling the respective physiological signals.

14. The method of claim 13, wherein approximating each physiological signal from the sampled data comprises, using the respective sensing matrix, solving for a sparse representation of the physiological signal by applying compressive sensing to the respective sampled data.

15. The method as claimed in claim 13 or 14, wherein updating each sensing matrix comprises determining a respective basis that sparsely represents the respective approximated signal and determining a respective updated sensing matrix that is incoherent with the determined basis.

**Patentansprüche**

1. System (1) zum adaptiven Erfassen physiologischer Daten unter Verwendung von Compressed Sensing, wobei das System umfasst:

   eine Vielzahl von Sensoren (2), die jeweils so konfiguriert sind, dass sie ein jeweiliges physiologisches Signal gemäß einem Abtastmuster (5) abtasten, das durch eine jeweilige Erfassungsmatrix definiert ist; und
   einen Prozessor (3), der so konfiguriert ist, dass er:

   abgetastete Daten von der Vielzahl von Sensoren (2) empfängt;
   physiologische Signale aus den abgetasteten Daten approximiert;
   eine oder mehrere Korrelationen zwischen den approximierten physiologischen Signalen identifiziert;
   eine oder mehrere der Erfassungsmatrizen in Abhängigkeit von der einen oder den mehreren erkannten Korrelationen aktualisiert; und
   eines oder mehrere der aktualisierten Abtastmuster (5), die durch die aktualisierten Erfassungsmatrizen definiert sind, an die jeweiligen Sensoren der Vielzahl von Sensoren (2) zur Verwendung beim Abtasten der jeweiligen physiologischen Signale überträgt.

2. System wie in Anspruch 1 beansprucht, wobei der Prozessor so konfiguriert ist, dass er jedes physiologische Signal aus den abgetasteten Daten durch Lösen nach einer schwach besetzten Darstellung des physiologischen Signals unter Verwendung der jeweiligen Erfassungsmatrix durch Anwenden von Compressed Sensing auf die jeweiligen abgetasteten Daten approximiert.

3. System wie in einem der vorhergehenden Ansprüche beansprucht, wobei der Prozessor so konfiguriert ist, dass er jedes physiologische Signal aus den abgetasteten Daten unter Verwendung von Interpolation oder Hauptkomponentenanalyse approximiert.

4. System wie in einem der vorhergehenden Ansprüche beansprucht, wobei der Prozessor so konfiguriert ist, dass er jede Erfassungsmatrix aktualisiert, indem er eine jeweilige Basis bestimmt, die das jeweilige approximierte Signal schwach besetzt darstellt, und eine jeweilige aktualisierte Erfassungsmatrix bestimmt, die zu der bestimmten Basis inkohärent ist.

5. System wie in Anspruch 4 beansprucht, wenn abhängig von Anspruch 2, wobei das Lösen für eine schwach besetzte Darstellung jedes physiologischen Signals unter Verwendung der jeweiligen zuvor bestimmten Basis durchgeführt wird, um das zuvor approximierte physiologische Signal schwach besetzt darzustellen.

6. System wie in Anspruch 4 oder 5 beansprucht, wobei der Prozessor so konfiguriert ist, dass er Änderungen an dem jeweiligen physiologischen Signal basierend auf einem Änderungsmaß der jeweiligen Basis jedes Mal ableitet, wenn die jeweilige Erfassungsmatrix aktualisiert wird.

7. System wie in einem der Ansprüche 4 bis 6 beansprucht, wobei für jedes physiologische Signal die jeweilige Basis unter Verwendung eines Sparse-Coding-Algorithmus bestimmt wird, der eine schwächer besetzte Basis findet, mit der das jeweilige approximierte Signal dargestellt wird, wobei das approximierte Signal als Trainingsdaten für den Sparse-Coding-Algorithmus verwendet wird.

8. System wie in Anspruch 7 beansprucht, wobei die identifizierten Korrelationen als zusätzliche Einschränkung in dem Sparse-Coding-Algorithmus verwendet werden.

9. System wie in einem der vorhergehenden Ansprüche beansprucht, wenn abhängig von Anspruch 2, wobei das Compressed Sensing unter Verwendung einer $l_1$-Norm angewendet wird, um die schwach besetzte Darstellung des physiologischen Signals zu lösen.

10. System wie in einem der vorhergehenden Ansprüche beansprucht, wobei der Prozessor ein Maß von Korrelation zwischen zwei oder mehr physiologischen Signalen identifiziert.

11. System wie in Anspruch 10 beansprucht, wobei der Prozessor eine oder mehrere Erfassungsmatrizen in Abhängigkeit davon aktualisiert, ob das Maß von Korrelation zwischen zwei oder mehr physiologischen Signalen von einem erwarteten Wert um mehr als einen vordefinierten Schwellenwert abweicht, oder in Abhängigkeit davon, ob die Änderungsrate des Maßes von Korrelation zwischen zwei oder mehr physiologischen Signalen von einem erwarteten Wert um mehr als einen vordefinierten Schwellenwert abweicht.

12. System nach einem der vorhergehenden Ansprüche, wobei die physiologischen Signale schwach besetzte Signale sind, sodass jedes physiologische Signal der Länge N als eine lineare Kombination von K Basisvektoren dargestellt werden kann, wobei K << N.

13. Verfahren zum adaptiven Erfassen physiologischer Daten unter Verwendung von Compressed Sensing, wobei das Verfahren umfasst:

Abtasten (S210) physiologischer Signale an einer Vielzahl von Sensoren, wobei jeder Sensor so konfiguriert ist, dass er ein jeweiliges physiologisches Signal gemäß einem jeweiligen Abtastmuster abtastet, das durch eine jeweilige Erfassungsmatrix definiert ist; und
Approximieren (S230) der physiologischen Signale aus den von den Sensoren abgetasteten Daten;
Identifizieren (S235) einer oder mehrerer Korrelationen zwischen den approximierten physiologischen Signalen;
Aktualisieren (S250) einer oder mehreren der Erfassungsmatrizen in Abhängigkeit von der einen oder den mehreren identifizierten Korrelationen; und
Übertragen (S260) eines oder mehrere der aktualisierten Abtastmuster, die durch die jeweiligen aktualisierten Erfassungsmatrizen definiert sind, an jeweilige Sensoren zur Verwendung beim Abtasten der jeweiligen physiologischen Signale.

14. Verfahren nach Anspruch 13, wobei das Annähern jedes physiologischen Signals aus den abgetasteten Daten unter Verwendung der jeweiligen Erfassungsmatrix das Lösen einer schwach besetzten Darstellung des physiologischen Signals durch Anwenden von Compressed Sensing auf die jeweiligen abgetasteten Daten umfasst.

15. Verfahren wie in Anspruch 13 oder 14 beansprucht, wobei das Aktualisieren jeder Erfassungsmatrix das Bestimmen einer jeweiligen Basis, die das jeweilige approximierte Signal schwach besetzt darstellt, und das Bestimmen einer jeweiligen aktualisierten Erfassungsmatrix, die zu der bestimmten Basis inkohärent ist, umfasst.

## Revendications

1. Système (1) de capture adaptative de données physiologiques au moyen d'une détection par compression, le système comprenant :

une pluralité de capteurs (2) configurés chacun pour échantillonner un signal physiologique respectif selon un modèle d'échantillonnage (5) défini par une matrice de détection respective ; et
un processeur (3) configuré pour :

recevoir des données échantillonnées en provenance de la pluralité de capteurs (2) ;
approximer les signaux physiologiques à partir des données échantillonnées ;
identifier une ou plusieurs corrélations entre les signaux physiologiques approchés ;
mettre à jour une ou plusieurs des matrices de détection en fonction de la ou des corrélations détectées ; et
émettre un ou plusieurs modèles d'échantillonnage (5) mis à jour définis par les matrices de détection mises à jour vers les capteurs respectifs de la pluralité de capteurs (2) pour une utilisation dans l'échantillonnage des signaux physiologiques respectifs.

2. Système selon la revendication 1, ledit processeur étant configuré pour approximer chaque signal physiologique à partir des données échantillonnées en résolvant, au moyen de la matrice de détection respective, une représentation éparse du signal physiologique en appliquant une détection par compression aux données échantillonnées respectives.

3. Système selon l'une quelconque des revendications précédentes, ledit processeur étant configuré pour approximer chaque signal physiologique à partir des données échantillonnées au moyen d'une interpolation ou d'une analyse en composantes principales.

4. Système selon l'une quelconque des revendications précédentes, ledit processeur étant configuré pour mettre à jour chaque matrice de détection en déterminant une base respective qui représente de manière éparse le signal approché respectif et déterminer une matrice de détection mise à jour respective qui est incohérente avec la base déterminée.

5. Système selon la revendication 4 lorsqu'elle dépend de la revendication 2, ladite résolution d'une représentation éparse de chaque signal physiologique étant réalisée au moyen de la base respective précédemment déterminée pour représenter de manière éparse le signal physiologique précédemment approché.

6. Système selon la revendication 4 ou 5, ledit processeur étant configuré pour déduire des changements du signal physiologique respectif sur la base d'une mesure de changement de la base respective chaque fois que la matrice de détection respective est mise à jour.

7. Système selon l'une quelconque des revendications 4 à 6, pour chaque signal physiologique, ladite base respective étant déterminée au moyen d'un algorithme de codage épars qui trouve une base plus éparse avec laquelle représenter le signal approché respectif, ledit signal approché étant utilisé en tant que données de formation pour l'algorithme de codage épars.

8. Système selon la revendication 7, lesdites corrélations identifiées étant utilisées en tant que contrainte supplémentaire dans l'algorithme de codage épars.

9. Système selon l'une quelconque des revendications précédentes lorsqu'elle dépend de la revendication 2, ladite détection par compression étant appliquée au moyen d'une norme $l_1$ de façon à résoudre la représentation éparse du signal physiologique.

10. Système selon l'une quelconque des revendications précédentes, ledit processeur identifiant une mesure de corrélation entre deux signaux physiologiques ou plus.

11. Système selon la revendication 10, ledit processeur mettant à jour une ou plusieurs matrices de détection en fonction du fait que la mesure de corrélation entre deux signaux physiologiques ou plus s'écarte ou non d'une valeur attendue de plus d'un seuil prédéfini, ou en fonction du fait que le taux de changement de la mesure de corrélation entre deux signaux physiologiques ou plus s'écarte ou non d'une valeur attendue de plus d'un seuil prédéfini.

12. Système selon l'une quelconque des revendications précédentes, lesdits signaux physiologiques étant des signaux épars de sorte que chaque signal physiologique de longueur N puisse être représenté comme une combinaison linéaire de K vecteurs de base, où K << N.

13. Procédé permettant la capture adaptative de données physiologiques au moyen d'une détection par compression, le procédé comprenant :

l'échantillonnage (S210) de signaux physiologiques au niveau d'une pluralité de capteurs, chaque capteur étant configuré pour échantillonner un signal physiologique respectif selon un modèle d'échantillonnage respectif défini par une matrice de détection respective ; et
l'approximation (S230) des signaux physiologiques à partir de données échantillonnées par les capteurs ;
l'identification (S235) d'une ou plusieurs corrélations entre les signaux physiologiques approchés ;
la mise à jour (S250) d'une ou plusieurs des matrices de détection en fonction de la ou des corrélations identifiées ; et
l'émission (S260) d'un ou plusieurs modèles d'échantillonnage mis à jour définis par les matrices de détection mises à jour respectives vers des capteurs respectifs pour une utilisation dans l'échantillonnage des signaux physiologiques respectifs.

14. Procédé de la revendication 13, ladite approximation de chaque signal physiologique à partir des données échantillonnées comprenant, au moyen de la matrice de détection respective, la résolution d'une représentation éparse du signal physiologique en appliquant une détection par compression aux données échantillonnées respectives.

15. Procédé selon la revendication 13 ou 14, ladite mise à jour de chaque matrice de détection comprenant la détermination d'une base respective qui représente de manière éparse le signal approché respectif et la détermination d'une matrice de détection mise à jour respective qui est incohérente avec la base déterminée.

Figure 1

Figure 2a

Figure 2b

$$y \qquad \Phi \qquad x \qquad \Phi \qquad \Psi \qquad s$$

$$\begin{bmatrix} y_1 \\ y_2 \\ \cdots \\ y_M \end{bmatrix} = \begin{bmatrix} 1 & 0 & \cdots & 0 \\ \cdots & 1 & 0 & \cdots \\ 0 & \cdots & 0 & 0 \end{bmatrix} \begin{bmatrix} x_1 \\ \cdots \\ x_M \\ \cdots \\ x_N \end{bmatrix} = \begin{bmatrix} 1 & 0 & \cdots & 0 \\ \cdots & 1 & 0 & \cdots \\ 0 & \cdots & 0 & 0 \end{bmatrix} \begin{bmatrix} \Psi_1 & \cdots & \Psi_N \\ \cdots & \cdots & \cdots \\ \cdots & \cdots & \cdots \end{bmatrix} \begin{bmatrix} s_1 \\ 0 \\ \cdots \\ 0 \\ s_K \\ \cdots \\ s_N \end{bmatrix}$$

$$D$$

Figure 3

Initialise the plurality of sensors using random sensing matrices
S100

Sample data according to the received sampling pattern at each sensor
S110

Transmit sampled data to the processor
S120

Recover original signals from the sampled data using compressive sensing
S125

Figure 4

Initialise the plurality of sensors
using random sensing matrices
S200

Sample data according to the
received sampling pattern at
S210                 each sensor

Transmit sampled data to the
S220                 processor

Approximate original signals
based on sampled data
S230

Using
interpolation
methods

S230b

Using
compressive
sensing

S230a

Recover original
signals from the
sampled data using
compressive sensing
S225

Identify one or more correlations
between sensors
S235

Update the basis used to
sparsely represent the original
signal based on identified
S240          correlation(s)

Determine an updated
sensing matrix in
dependence on identified
S250    correlation(s)

Transmit an updated
sampling pattern to the
plurality of sensors in
dependence on sensing
S260          matrix

Figure 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2019125271 A

**Non-patent literature cited in the description**

- **F. SANFILIPPO** ; **K. Y. PETTERSEN**. A Sensor Fusion Wearable health-monitoring System with Haptic Feedback. *2015 11th International Conference on Innovations in Information Technology (IIT)*, 2015, 262-266

- **E. CANDÈS** ; **J. ROMBERG** ; **T. TAO**. Robust uncertainty principles: Exact signal reconstruction from highly incomplete frequency information. *IEEE Trans. Inform. Theory*, February 2006, vol. 52 (2), 489-509
- **D. DONOHO**. Compressed sensing. *IEEE Trans. Inform. Theory*, April 2006, vol. 52 (4), 1289-1306